# EUROPÄISCHE PATENTSCHRIFT

(11) **EP 1 292 269 B1**
(45) Veröffentlichungstag und Bekanntmachung des Hinweises auf die Patenterteilung: **09.02.2005**
(21) Anmeldenummer: 01945274.7
(22) Anmeldetag: 13.06.2001
(51) Int. Cl.: A61K 7/13, C07D 471/04, C07D 241/38

(54) **MITTEL ZUM FÄRBEN VON KERATINHALTIGEN FASERN**
AGENT FOR DYEING KERATIN CONTAINING FIBERS
AGENT DE COLORATION DE FIBRES KERATINIQUES

(30) Priorität: 21.06.2000 DE 10029441
(43) Veröffentlichungstag der Anmeldung: 19.03.2003
(73) Patentinhaber: Henkel Kommanditgesellschaft auf Aktien, 40589 Düsseldorf-Holthausen (DE)
(72) Erfinder: OBERKOBUSCH, Doris, 40591 Düsseldorf (DE); HÖFFKES, Horst, 40595 Düsseldorf (DE); MÖLLER, Hinrich, 40789 Monheim (DE); MARTIN, Hans-Dieter, 40591 Düsseldorf (DE); GROSS, Wibke, 40549 Düsseldorf (DE)
(86) Internationale Anmeldenummer: PCT/EP2001/006691
(87) Internationale Veröffentlichungsnummer: WO 2001/097765

(56) Entgegenhaltungen:
- WO-A-99/51688
- US-A- 5 089 025
- DATABASE CHEMICAL ABSTRACTS [Online] STN; access number 113: 201244, XP002179188 & JP 02 101451 A (FUJI PHOTO FILM CO., LTD) 13. April 1990 (1990-04-13)

## Beschreibung

Die Erfindung betrifft ein Mittel zum Färben von keratinhaltigen Fasern, insbesondere menschlichen Haaren, das mindestens ein Cyclopentachinoxaliniumderivat enthält, die Verwendung dieser Verbindungen in Mitteln zum Färben von keratinhaltigen Fasern sowie ein Verfahren zum Färben von keratinhaltigen Fasern, insbesondere menschlichen Haaren.

Für das Färben von keratinhaltigen Fasern, z. B. Haaren, Wolle oder Pelzen, kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Kuppler- und Entwicklerkomponenten werden auch als Oxidationsfarbstoffvorprodukte bezeichnet.

Als Entwicklerkomponenten werden üblicherweise primäre aromatische Amine mit einer weiteren, in para- oder ortho-Position befindlichen freien oder substituierten Hydroxyoder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazolon-5, 4-Amino-3-methylphenol, 2-Aminomethyl-4-aminophenol, 2-Hydroxymethyl-4-aminophenol, 2-Hydroxy-4,5,6-triaminopyrimidin, 2,4-Dihydroxy-5,6-diaminopyrimidin und 2,5,6-Triamino-4-hydroxypyrimidin.

Als Kupplerkomponenten werden in der Regel m-Phenylendiaminderivate, Naphthole, Resorcin und Resorcinderivate, Pyrazolone, m-Aminophenole und substituierte Pyridinderivate verwendet. Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmonomethylether, p-Phenylendiamin, 2,4-Diaminophenoxyethanol, 2-Amino-4-(2-hydroxyethylamino)-anisol (Lehmanns Blau), 1-Phenyl-3-methyl-pyrazolon-5, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin, 3-Amino-6-methoxy-2-methylamino-pyridin und 3,5-Diamino-2,6-dimethoxypyridin.

Bezüglich weiterer üblicher Farbstoffkomponenten wird ausdrücklich auf die Reihe "Dermatology", herausgeben von Ch. Culnan, H. Maibach, Verlag Marcel Dekker Inc., New York, Basel, 1986, Bd. 7, Ch. Zviak, The Science of Hair Care, Kap. 7, Seiten 248 - 250 (Direktziehende Farbstoffe), und Kap. 8, Seiten 264 - 267 (Oxidationsfarbstoffe), sowie das "Europäische Inventar der Kosmetikrohstoffe", 1996, herausgegeben von der Europäischen Kommission, erhältlich in Diskettenform vom Bundesverband der deutschen Industrie- und Handelsunternehmen für Arzneimittel, Reformwaren und Körperpflegemittel e.V., Mannheim, Bezug genommen.

Mit Oxidationsfarbstoffen lassen sich zwar intensive Färbungen mit guten Echtheitseigenschaften erzielen, die Entwicklung der Farbe geschieht jedoch i.a. unter dem Einfluss von Oxidationsmitteln wie z. B. H₂O₂, was in einigen Fällen Schädigungen der Faser zur Folge haben kann. Desweiteren können einige Oxidationsfarbstoffvorprodukte bzw. bestimmte Mischungen von Oxidationsfarbstoffvorprodukten bisweilen bei Personen mit empfindlicher Haut sensibilisierend wirken. Direktziehende Farbstoffe werden unter schonenderen Bedingungen appliziert, ihr Nachteil liegt jedoch darin, dass die Färbungen häufig nur über unzureichende Echtheitseigenschaften verfügen.

Die Verwendung der unten näher beschriebenen Cyclopentachinoxaliniumderivate zum Färben von keratinhaltigen Fasern ist bislang nicht bekannt.

Aufgabe der vorliegenden Erfindung ist es, Färbemittel für Keratinfasem, insbesondere menschliche Haare, bereitzustellen, die hinsichtlich der Farbtiefe, der Grauabdeckung und den Echtheitseigenschaften qualitativ den üblichen Oxidationshaarfärbemitteln mindestens gleichwertig sind, ohne jedoch unbedingt auf Oxidationsmittel wie z. B. H₂O₂ angewiesen zu sein. Darüber hinaus dürfen die Färbemittel kein oder lediglich ein sehr geringes Sensibilisierungspotential aufweisen.

Überraschenderweise wurde nun gefunden, dass die in der Formel I dargestellten Cyclopentachinoxaliniumderivate sich auch in Abwesenheit von oxidierenden Agentien hervorragend zum Färben von keratinhaltigen Fasern eignen. Sie ergeben Ausfärbungen mit hervorragender Brillanz und Farbtiefe und führen zu vielfältigen Farbnuancen. Der Einsatz von oxidierenden Agentien soll dabei jedoch nicht prinzipiell ausgeschlossen werden.

Gegenstand der Erfindung ist ein Mittel zum Färben von keratinhaltigen Fasern, insbesondere menschlichen Haaren, enthaltend mindestens ein Cyclopentachinoxaliniumderivat mit der Formel I, 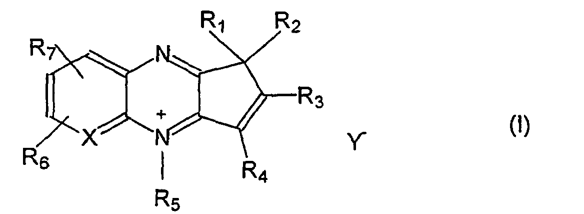 in der bedeuten
R¹, R², R³, R⁴, die jeweils gleich oder verschieden sein können, Wasserstoff oder C₁-C₄-Alkylgruppen,
R⁵ ein C₁-C₄-Alkyl-, ein Ar-C₁-C₄-alkyl-, Aryl-, C₂-C₄-Alkenyl-, C₁-C₄-Hydroxyalkyl- oder C₁-C₄-Carboxyalkylrest,
X eine Gruppe CH- oder ein Stickstoffatom
R⁶ und R⁷, die jeweils gleich oder verschieden sein können, Halogen-, eine C₁-C₄-Hydroxyl-, Amino-, C₁-C₄-Alkylamino-, C₁-C₄-Alkoxy-, Nitro-, C₁-C₄-Alkylgruppe, Wasserstoff, eine -COOH- oder -SO₃H-Gruppe,
Y⁻ Halogenid, Benzolsulfonat, p-Toluolsulfonat, Methansulfonat, Methylsulfat, Ethylsulfat, Trifluormethansulfonat, Perchlorat, Sulfat, Hydrogensulfat, Tetrafluorborat, oder Tetrachlorzinkat.

Unter keratinhaltigen Fasem sind Wolle, Pelze, Federn und insbesondere menschliche Haare zu verstehen. Die erfindungsgemäßen Färbemittel können prinzipiell aber auch zum Färben anderer Naturfasern, wie z. B. Baumwolle, Jute, Sisal, Leinen oder Seide, modifizierter Naturfasern, wie z. B. Regeneratcellulose, Nitro-, Alkyl- oder Hydroxyalkyloder Acetylcellulose und synthetischer Fasem, wie z. B. Polyamid-, Polyacrylnitril-, Polyurethan- und Polyesterfasem verwendet werden.

Die Verbindungen mit der Formel I sind zum großen Teil literaturbekannt, im Handel erhältlich oder nach bekannten Syntheseverfahren herstellbar.

Vorzugsweise sind die Verbindungen mit der Formel I ausgewählt aus 5-Aza-6-methoxy-1,1,3,4-tetramethyl-1H-cyclopenta(b)chinoxalinium-, 5-Aza-6-methoxy-1,1,2,3,4-pentamethyl-1H-cyclopenta(b)chinoxalinium-, 5-Aza-1,1,2,3,4-pentamethyl-1H-cyclopenta(b)-chinoxalinium-, 1,1,2,3-Tetramethyl-1H-cyclopenta(b)chinoxalinium-, 4-Phenyl-1,1,2,3-tetramethyl-1H-cyclopenta(b)chinoxalinium-, 7-Chlor-4-(2-propyl)-1,1,2,3-tetramethyl-1Hcyclopenta(b)chinoxalinium-, 7-Chlor-4-ethyl-1,1,2,3-tetramethyl-1H-cyclopenta(b)chinoxalinium-, 1,1,2,3,4-Pentamethyl-1H-cyclopenta(b)chinoxalinium-halogeniden, wie - chloriden,
-bromiden und -iodiden, -benzolsulfonaten, p-toluolsulfonaten, -methansuffonaten, -methylsulfaten, -ethylsulfaten, -trifluormethansulfonaten, -perchloraten, -sulfaten, -hydrogensulfaten, -tetrafluorboraten, -tetrachlorzinkaten sowie deren beliebigen Gemischen.

Die voranstehend genannten Cyclopentachinoxaliniumderivate mit der Formel I werden vorzugsweise in den erfindungsgemäßen Mitteln in einer Menge von 0,03 bis 65 mmol, insbesondere von 1 bis 40 mmol, bezogen auf 100 g des gesamten Färbemittels, verwendet. Sie können als direktziehende Färbemittel oder in Gegenwart von üblichen Oxidationsfarbstoffvorprodukten eingesetzt werden.

Färbemittel, die als färbende Komponente Cyclopentachinoxaliniumderivate allein enthalten, werden bevorzugt für hellere Färbungen eingesetzt. Färbungen mit noch erhöhter Brillanz und insbesondere Färbungen, die auf dem Haar eine Fluoreszens zeigen, können erhalten werden, wenn die Verbindungen mit der Formel I mit geeigneten aromatischen/heteroaromatischen Aldehyden und/oder Ketonen (Komponente B) verwendet werden.

Besonders geeignete Beispiele für aromatische/heteroaromatische Aldehyde und Ketone sind Salicylaldehyd, 3-Hydroxybenzaldehyd, 4-Hydroxybenzaldehyd, o-Anisaldehyd, m-Anisaldehyd, p-Anisaldehyd, 4-Hydroxy-3-methylbenzaldehyd, 2-Hydroxy-3-methylbenzaldehyd, 2-Hydroxy-5-methylbenzaldehyd, 2-Hydroxy-4-methylbenzaldehyd, 2,3-Dihydroxybenzaldehyd, 2,5- Dihydroxybenzaldehyd, 3,4- Dihydroxybenzaldehyd, 2,4- Dihydroxybenzaldehyd, 3,5- Dihydroxybenzaldehyd, 2,3-Dihydrobenzo[b]furan-5-carboxaldehyd, Piperonal, 4-Ethoxy-benzaldehyd, 3,5-Dimethyl-4-hydroxybenzaldehyd, Vanillin, Isovanillin, 2,3,4-Trihydroxybenzaldehyd, 2,4,5-Trihydroxybenzaldehyd, 2,4,6-Trihydroxybenzaldehyd, 3,4,5-Trihydroxybenzaldehyd, 3-Chlor-4-hydroxybenzaldehyd, 2,4-Dimethoxybenzaldehyd, 2,5-Dimethoxybenzaldehyd, 2,6-Dimethoxybenzaldehyd, 3,4-Dimethoxybenzaldehyd, 3,4-Dihydroxy-5-methoxybenzaldehyd, 2-Hydroxy-1-naphthaldehyd, 4-Hydroxy-1-naphthaldehyd, 1-Hydroxy-2-naphthaldehyd, 2-Methoxy-1-naphthaldehyd, 4-Methoxy-1-naphthaldehyd, 2-Hydroxyacetophenon, 4-Hydroxyacetophenon, 2,4-Dihydroxyacetophenon, 2-Hydroxyzimtaldehyd, 4-Hydroxyzimtaldehyd, 2,4-Dihydroxyzimtaldehyd, 4-Hydroxy-benzylidenaceton, 4-Hydroxy-3-methoxybenzylidenaceton, 4-Hydroxy-3-methoxyzimtaldehyd (Coniferylaldehyd), 3,5-Dimethoxy-4-hydroxyzimtaldehyd, 4-Hydroxycinnamylidenacetaldehyd, 4-Methoxycinnamylidenacetaldehyd sowie beliebige Gemische der voranstehenden.

In allen Färbemitteln können auch mehrere verschiedene Cyclopentachinoxaliniumderivate der Formel I gemeinsam zum Einsatz kommen; ebenso können auch mehrere verschiedene aromatische Aldehyde und Ketone der Komponente B gemeinsam verwendet werden.

Unter die voranstehend beschriebene Ausführungsform fällt auch die Verwendung von solchen Substanzen, die Reaktionsprodukte von Cyclopentachinoxaliniumderivaten der Formel I mit den genannten aromatischen/heteroaromatischen Aldehyden und Ketonen darstellen, als direktziehende Färbemittel. Derartige Reaktionsprodukte können z. B. durch kurzes Erwärmen der beiden Komponenten in stöchiometrischen Mengen in wässrigem neutralen bis schwach alkalischen Milieu erhalten werden, wobei sie entweder als Feststoff aus der Lösung ausfallen oder durch Eindampfen der Lösung daraus isoliert werden. Die Reaktionsprodukte können auch in Kombination mit anderen Farbstoffen oder Farbstoffvorprodukten eingesetzt werden.

Die voranstehend genannten Verbindungen der Komponente B können in einer Menge von jeweils 0,03 bis 65, insbesondere 1 bis 40 mmol, jeweils bezogen auf 100 g des gesamten Färbemittels, eingesetzt werden. Weiterhin kann es erfindungsgemäß besonders bevorzugt sein, die Verbindung der Formel I und die Verbindung der Komponente B im molaren Mengenverhältnis von 2 : 1 bis 1 : 2, insbesondere etwa äquimolar, einzusetzen.

Zur Erlangung weiterer und intensiverer Ausfärbungen können die erfindungsgemäßen Mittel zusätzlich Farbverstärker enthalten. Die Farbverstärker sind vorzugsweise ausgewählt aus der Gruppe bestehend aus Piperidin, Piperidin-2-carbonsäure, Piperidin-3-carbonsäure, Piperidin-4-carbonsäure, Pyridin, 2-Hydroxypyridin, 3-Hydroxypyridin, 4-Hydroxypyridin, Imidazol, 1-Methylimidazol, Histidin, Pyrrolidin, Prolin, Pyrrolidon, Pyrrolidon-5-carbonsäure, Pyrazol, 1,2,4-Triazol, Piperazidin, deren Derivate sowie deren physiologisch verträglichen Salzen.

Die voranstehend genannten Farbverstärker können in einer Menge von jeweils 0,03 bis 65, insbesondere 1 bis 40 mmol, jeweils bezogen auf 100 g des gesamten Färbemittels, eingesetzt werden.

Auf die Anwesenheit von Oxidationsmitteln, z. B. H₂O₂, kann dabei verzichtet werden. Es kann jedoch u. U. wünschenswert sein, den erfindungsgemäßen Mitteln zur Erzielung der Nuancen, die heller als die zu färbende keratinhaltige Faser sind, Wasserstoffperoxid oder andere Oxidationsmittel zuzusetzen. Oxidationsmittel werden in der Regel in einer Menge von 0,01 bis 6 Gew.-%, bezogen auf die Anwendungsmischung, eingesetzt. Ein für menschliches Haar bevorzugtes Oxidationsmittel ist H₂O₂.

In einer bevorzugten Ausführungsform enthalten die erfindungsgemäßen Färbemittel zur weiteren Modifizierung der Farbnuancen neben den erfindungsgemäß enthaltenen Verbindungen zusätzlich übliche direktziehende Farbstoffe, z. B. aus der Gruppe derNitrophenylendiamine, Nitroaminophenole, Anthrachinone oder Indophenole, wie z. B. die unter den internationalen Bezeichnungen bzw. Handelsnamen HC Yellow 2, HC Yellow 4, HC Yellow 6, Basic Yellow 57, Disperse Orange 3, HC Red 3, HC Red BN, Basic Red 76, HC Blue 2, Disperse Blue 3, Basic Blue 99, HC Violet 1, Disperse Violet 1, Disperse Violet 4, Disperse Black 9, Basic Brown 16 und Basic Brown 17 bekannten Verbindungen sowie Pikraminsäure 2-Amino-6-chloro-4-nitrophenol, 4-Amino-2-nitrodiphenylamin-2'carbonsäure, 6-Nitro-1,2,3,4-tetrahydrochinoxalin, 4-N-Ethyl-1,4-bis-(2'-hydroxyethylamino)-2-nitrobenzol-hydrochlorid und 1-Methyl-3-nitro-4-(2'-hydroxyethyl)-aminobenzol. Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe, wie sie beispielsweise in Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten sind, enthalten.

Es ist nicht erforderlich, dass die Oxidationsfarbstoffvorprodukte oder die fakultativ enthaltenen direktziehenden Farbstoffe jeweils einheitliche Verbindungen darstellen. Vielmehr können in den erfindungsgemäßen Färbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Die erfindungsgemäßen Färbemittel ergeben bereits bei physiologisch verträglichen Temperaturen von unter 45°C intensive Färbungen. Sie eignen sich deshalb besonders zum Färben von menschlichen Haaren. Zur Anwendung auf dem menschlichen Haar können die Färbemittel üblicherweise in einen wasserhaltigen kosmetischen Träger eingearbeitet werden. Geeignete wasserhaltige kosmetische Träger sind z. B. Cremes, Emulsionen, Gele oder auch tensidhaltige schäumende Lösungen wie z. B. Shampoos oder andere Zubereitungen, die für die Anwendung auf den keratinhaltigen Fasern geeignet sind. Falls erforderlich ist es auch möglich, die Färbemittel in wasserfreie Träger einzuarbeiten.

Weiterhin können die erfindungsgemäßen Färbemittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im MolekülGlykoloder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobemsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2 bis 15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quatemisierten Proteinhydrolysate dar.

Erfindungsgemäß ebenfalls geeignet sind kationische Silikonöle wie beispielsweise die im Handel erhältlichen Produkte Q2-7224 (Hersteller: Dow Coming; ein stabilisiertes Trimethylsilylamodimethicon), Dow Coming 929 Emulsion (enthaltend ein hydroxyl-aminomodifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quatemäre Esterverbindungen, sogenannte "Esterquats", wie die unter dem Warenzeichen Stepantex® vertriebenen Methylhydroxyalkyldialkoyloxyalkylammoniummethosulfate.

Ein Beispiel für ein als kationisches Tensid einsetzbares quatemäres Zuckerderivat stellt das Handelsprodukt Glucquat®100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quatemisierte Celluloseether, Polysiloxane mit quatemären Gruppen, Dimethyldiallylammoniumchlorid-Polymere, Acrylamid-Dimethyldiallylammoniumchlorid-Copolymere, mit Diethylsulfat quatemierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methylmethacrylat/tert.-Butylaminoethylmethacrylat/2-Hydroxypropylmethacrylat-Copolymere,
- anionische Polymere wie beispielsweise Polyacrylsäuren, vemetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.-Butylacrylamid-Terpolymere,
- Verdickungsmittel wie Agar-Agar, Guar-Gum, Alginate, Xanthan-Gum, Gummi arabicum, Karaya-Gummi, Johannisbrotkemmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Imidazole, Tannine, Pyrrol,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Bestandteile des wasserhaltigen Trägers werden zur Herstellung der erfindungsgemäßen Färbemittel in für diesen Zweck üblichen Mengen eingesetzt; z. B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Für das Färbeergebnis kann es vorteilhaft sein, den Färbemitteln Ammonium- oder Metallsalze zuzugeben. Geeignete Metallsalze sind z. B. Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, wobei Natriumacetat, Lithiumbromid, Calciumbromid, Calciumgluconat, Zinkchlorid, Zinksulfat, Magnesiumchlorid, Magnesiumsulfat, Ammoniumcarbonat, -chlorid und -acetat bevorzugt sind. Diese Salze sind vorzugsweise in einer Menge von 0,03 bis 65, insbesondere von 1 bis 40 mmol, bezogen auf 100 g des gesamten Färbemittels, enthalten.

Der pH-Wert der gebrauchsfertigen Färbezubereitungen liegt üblicherweise zwischen 2 und 11, vorzugsweise zwischen 5 und 10.

Ein weiterer Gegenstand der vorliegenden Erfindung betrifft die Verwendung von Cyclopentachinoxaliniumderivaten mit der Formel I, 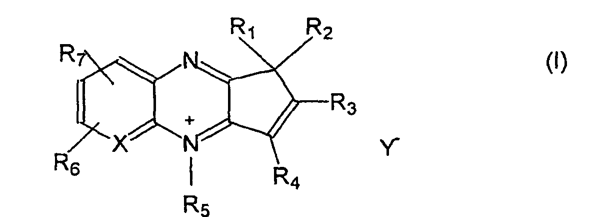 in der R¹, R², R³, R⁴, R⁵, R⁶, R⁷, X und Y⁻ wie oben definiert sind,
als eine färbende Komponente in Oxidationshaarfärbemitteln.

Noch ein weiterer Gegenstand der vorliegenden Erfindung betrifft ein Verfahren zum Färben von keratinhaltigen Fasern, insbesondere menschlichen Haaren, worin ein Färbemittel, enthaltend
A) mindestens ein Cyclopentachinoxaliniumderivat mit der Formel I, 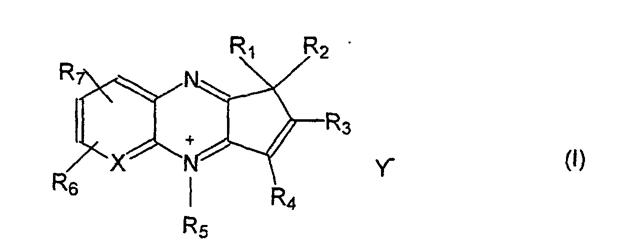 in der R¹, R², R³, R⁴, R⁵, R⁶, R⁷, X und Y⁻ wie oben definiert sind,
   und
B) einen oder mehrere aromatische Aldehyde und/oder Ketone,
sowie übliche kosmetische Inhaltsstoffe, auf die keratinhaltigen Fasern aufgebracht, einige Zeit, üblicherweise ca. 30 Minuten, auf der Faser belassen und anschließend wieder ausgespült oder mit einem Shampoo ausgewaschen wird.

Die Cyclopentachinoxaliniumderivate der Formel I und die aromatischen/heteroaromatischen Aldehyde und/oder Ketone können entweder gleichzeitig auf das Haar aufgebracht werden oder aber auch nacheinander, wobei es unerheblich ist, welche der beiden Komponenten zuerst aufgetragen wird. Die fakultativ enthaltenen Ammoniumoder Metallsalze können dabei der ersten oder der zweiten Komponente zugesetzt werden. Zwischen dem Auftragen der ersten und der zweiten Komponente können bis zu 30 Minuten Zeitabstand liegen. Auch eine Vorbehandlung der Fasern mit der Salzlösung ist möglich.

Die Cyclopentachinoxaliniumderivate der Formel I und die aromatischen/heteroaromatischen Aldehyde und/oder Ketone können entweder getrennt oder zusammen gelagert werden, entweder in einer flüssigen bis pastösen Zubereitung (wässrig oderwasserfrei) oder als trockenes Pulver. Werden die Komponenten in einer flüssigen Zubereitung zusammen gelagert, so sollte diese zur Verminderung einer Reaktion der Komponenten weitgehend wasserfrei sein. Bei der getrennten Lagerung werden die reaktiven Komponenten erst unmittelbar vor der Anwendung miteinander innig vermischt. Bei der trockenen Lagerung wird vor der Anwendung üblicherweise eine definierte Menge warmen (30 bis 80°C) Wassers hinzugefügt und eine homogene Mischung hergestellt.

Einige der Verbindungen der Formel I sind neu. Noch ein weiterer Gegenstand der vorliegenden Erfindung sind demgemäß die Salze, insbesondere die Halogenide, wie Chloride, Bromide und lodide, Benzolsulfonate, p-Toluolsulfonate, Methansulfonate, Methylsulfate, Ethylsulfate, Trifluormethansulfonate, Perchlorate, Sulfate, Hydrogensulfate, Tetrafluorborate, Tetrachlorzinkate, von 5-Aza-6-methoxy-1,1,3,4-tetramethyl-1H-cyclopenta(b)chinoxalinium und 5-Aza-6-methoxy-1,1,2,3,4-pentamethyl-1H-cyclopenta(b)chinoxalinium.

### Beispiele

### Herstellung der Färbekomponenten

### Beispiel 1: Darstellung von 5-Aza-6-methoxy-1,1,2,3,4,-pentamethyl-1H-cyclopenta(b)-chinoxaliniumtetrafluoroborat (6-Methoxy-1,1,2,3,4-pentamethyl-4a,8a-dihydro-1H-4,5,9-triaza-cyclopenta-(b)naphtalen)

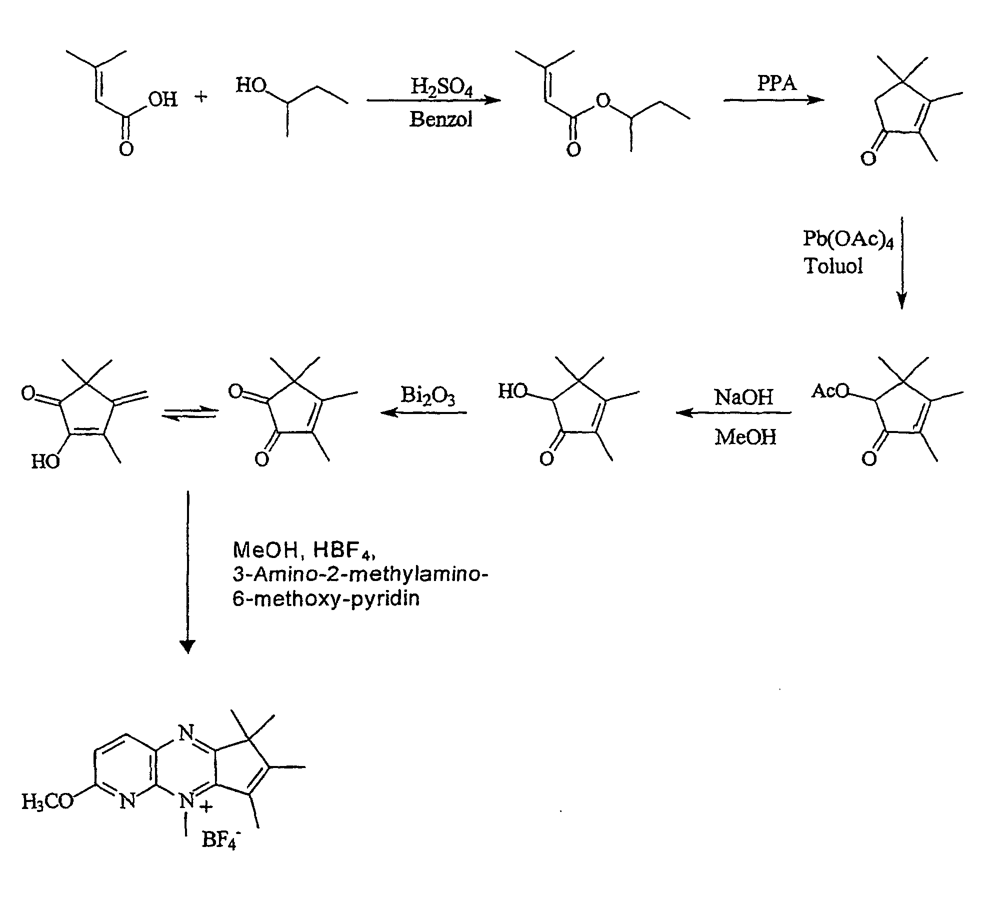

### 1. Stufe: Darstellung von 3-Methyl-2-butensäure-(2'-butylester)

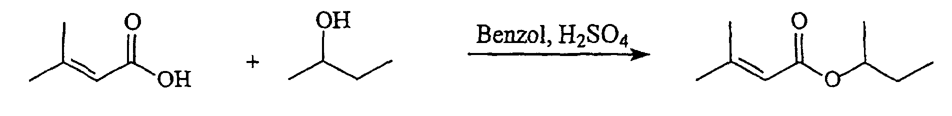

In einem mit Wasserabscheider versehenen 2 I Einhalskolben wurden 200 g (2,0 mol) käufliche 3,3-Dimethylacrylsäure und 148 g (2,0 mol) Isobutanol in 540 ml Benzol gelöst, 7 ml konzentrierte Schwefelsäure zugegeben und solange unter Rückfluß erhitzt, bis sich kein Reaktionswasser mehr abschied (ca. 72 Stunden). Das erkaltete Reaktionsgemisch wurde zweimal mit 100 ml gesättigter Natriumchloridlösung und einmal mit 100 ml gesättigter Natriumhydrogencarbonatlösung gewaschen. Nach dem Trocknen über Magnesiumsulfat wurde das Lösungsmittel am Rotationsverdampfer entfernt und der hellgelbe Rückstand im Wasserstrahlvakuum destilliert. Man erhielt das Produkt bei einer Übergangstemperatur von 68°C (13 Torr) als farblose Flüssigkeit.
- Ausbeute:: 235,3 g (1,5 mol) 77% der Theorie
- Sdp.: 68°C (13 Torr)
- n_{D}²⁰:: 1,4383

### 2. Stufe: Darstellung von 2,3,4,4-Tetramethyl-cyclopent-2-en-1-on

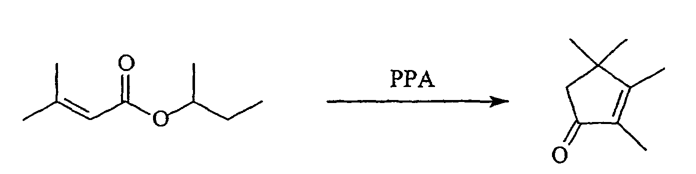

Unter kräftigem Rühren wurden 1 kg Polyphosphorsäure (PPA) in einem mit Rückflußkühler, KPG-Rührer mit Stahlrührwelle und Tropftrichter bestückten 2 I Dreihalskolben auf 120°C Ölbadtemperatur erwärmt. Innerhalb von 30 Minuten wurden 200,0 g (1,28 mol) 3-Methyl-2-butensäure-(2'-butylester) hinzugetropft und anschließend noch eine Stunde nachgerührt. Das Heizbad wurde entfernt und der noch heiße Kolben bei abgestelltem Rührer mit gestoßenem Eis auf 2/3 seines Volumens aufgefüllt. Es wurde wieder angerührt und mit Eiswasser auf ca. 1,7 I aufgefüllt. Die Lösung wurde mit festem Ammoniumchlorid gesättigt und anschließend fünfmal mit je 200 ml Diethylether extrahiert. Die vereinigten Etherphasen wurden mit je 200 ml zehnprozentiger Natriumhydrogencarbonatlösung und gesättigter Natriumchloridlösung gewaschen. Nach Trocknen über Magnesiumsulfat und Entfemen des Lösungsmittels am Rotationsverdampfer wurde der dunkelbraune Rückstand fraktioniert destilliert und das Produkt als hellgelbe Flüssigkeit bei einer Übergangstemperatur von 70 °C (5 Torr) erhalten.
- Ausbeute:: 87,4 g (0,63 mol), 49% der Theorie
- Sdp.:: 70°C (5 Torr)
- n_{D}²⁰:: 1,4727

### 3. Stufe: Darstellung von 5-Acetoxy-2,3,4,4-tetramethyl-2-cyclopenten-1-on

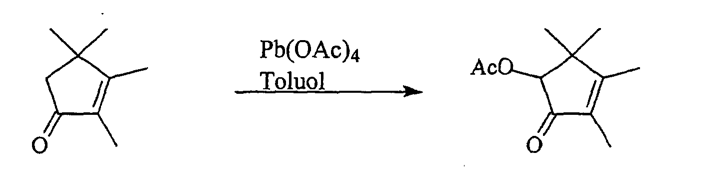

Unter Stickstoffatmosphäre wurden in einem 2 I Dreihalskolben 385 g (0,87 mol) Blei(IV)-acetat in 1,3 I absolutem Toluol vorgelegt und unter Rühren mit einem KPG-Rührer erwärmt. Bei einer Temperatur von 80°C wurden rasch 100 g (0,73 mol) 2,3,4,4,-Tetramethyl-cyclopent-2-en-1-on zum Oxidationsmittel zugetropft und anschließend sechs Stunden unter Rückfluß erhitzt. Das voluminös ausfallende Blei(II)acetat wurde noch heiß über einen Büchnertrichter abfiltriert, und der Filterrückstand wurde bis zur Farblosigkeit mehrfach mit Diethylether ausgewaschen. Die vereinigten organischen Filtrate wurden zweimal mit je 200 ml gesättigter Natriumchloridlösung gewaschen, die wässrige Phase wurde zweimal mit je 200 ml Diethylether extrahiert. Nach Trocknen über Magnesiumsulfat wurde das Lösungsmittel am Rotationsverdampfer abgezogen und der ölige Rückstand im Ölpumpenvakuum über eine 30 cm Vigreuxkolonne destilliert. Bei Übergangstemperaturen bis zu 60°C (0,8 Torr) wurde nicht umgesetztes Cyclopentenon zurückgewonnen. Man erhielt das Acyloinacetat bei einer Übergangstemperatur von 93 - 96°C (1 Torr).
- Ausbeute:: 72 g , 49% der Theorie

### 4. Stufe: Darstellung von 5-Hydroxy-2,3,4,4-tetramethyl-2-cyclopenten-1-on

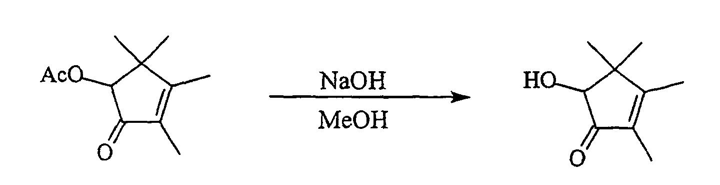

Eine Lösung von 60,0 g (0,30 mol) 5-Acetoxy-2,3,4,4-tetramethyl-2-cyclopenten-1-on in 400 ml Methanol wurde unter Stickstoffatmosphäre in einem 2 IDreihalskolben vorgelegt und mit Eis gekühlt. In der Kälte tropfte man innerhalb von 20 Minuten 300 ml 1 N Natriumhydroxidlösung zu und rührte das Reaktionsgemisch weitere zwei Stunden bei Raumtemperatur. Durch Zugabe von 70 ml 0,5 N Schwefelsäurelösung wurde das Reaktionsgemisch auf einen pH-Wert von ca. 6 gebracht. Es wurde mit 500 ml gesättigter Natriumchloridlösung versetzt und schließlich viermal mit je 300 ml Diethylether extrahiert. Die vereinigten organischen Phasen wurden mit 100 ml gesättigter Natriumchloridlösung gewaschen und getrocknet über Magnesiumsulfat. Nach Abziehen des Lösungsmittels erhielt man ein braungelbes Öl, welches im Ölpumpenvakuum über eine 30 cm Vigreuxkolonne destilliert wurde. Bei einer Übergangstemperatur von 62 - 64°C (0,4 Torr) erhielt man das Produkt als gelbes Öl.
- Ausbeute:: 32 g (207 mmol), 68% der Theorie
- Sdp.:: 62 - 64°C (0,4 Torr)

### 5. Stufe: Darstellung von 3,4,5,5-Tetramethylcyclopent-3-en-1,2-dion

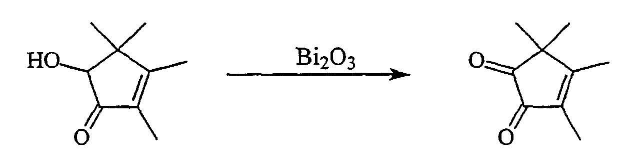

Eine Lösung von 30 g (0,20 mol) 5-Hydroxy-2,3,4,4-tetramethyl-2-cyclopenten-1-on in 200 ml Eisessig wurde in einem 500 ml Rundkolben auf 80°C erwärmt, bevor man sie unter Rühren mit 65 g (170 mmol) Wismut(III)oxid versetzte. Unter Stickstoffatmosphäre wurde das Reaktionsgemisch nun weitere acht Stunden unter Rückfluß erhitzt. Die mit der Abscheidung elementaren Wismuts einhergehende Trübung des Ansatzes zeigte den Umsatz des Oxidans an. Nach Beendigung der Reaktion wurde noch heiß vom ausgefallenen grauen Niederschlag abgesaugt und der Filterrückstand mit Eisessig nachgewaschen. Die Filtrate wurden am Rotationsverdampfer scharf eingeengt; der anfallende ölige Rückstand wurde mit 700 ml Diethylether aufgekocht. Nach Abnutschen vom unlöslichen Wismut(III)acetat wurde das Filtrat erneut vom Lösungsmittel befreit und im Ölpumpenvakuum fraktioniert destilliert. Bei einer Übergangstemperatur von 73°C (0,6 Torr) erhielt man das Diketon als intensiv oranges Öl, welches schon am Kühlfinger in seiner farblosen Enolform auskristallisieren kann.
- Ausbeute:: 25 g (164 mmol), 84% der Theorie
- Sdp.:: 73°C (0,6 Torr)

### Stufe 6: Darstellung von 5-Aza-6-methoxy-1,1,2,3,4-pentamethyl-1H-cyclopenta(b)-chinoxaliniumtetrafluoroborat (6-Methoxy-1,1,2,3,4-pentamethyl-4a,8a-dihydro-1H-4,5,9-triaza-cyclopenta-(b)naphtalen)

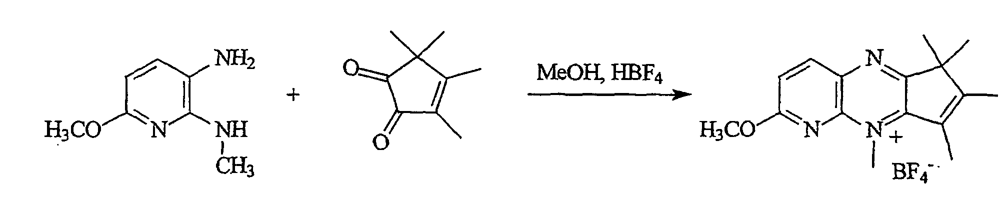

Es wurden 2,00 g (8,8 mmol) 3-Amino-2-methylamino-6-Methoxy-pyridin*2HCl zusammen mit 1,36 g (8,8 mmol) 3,4,5,5-Tetramethyl-cyclopent-2-en-1,2-dion in 50 ml Methanol und 9 ml 32%iger Tetrafluorborsäure 6 Stunden bei Raumtemperatur gerührt. Der ausgefallene gelbbraune Niederschlag wurde über einen Büchnertrichter abfiltriert und mit wenig Methanol gewaschen. Es wurde zweimal in Aceton umkristallisiert.
- Ausbeute:: 1,2 g (4,43 mmol), 50% der Theorie

### Beispiel 2: Darstellung von 5-Aza-6-methoxy-1,1,3,4-tetramethyl-1H-cyclopenta(b)-chinoxaliniumtetrafluoroborat

### 1. Stufe: Darstellung von 3,5,5-Trimethylcyclopent-3-en-1,2-dion

In einem 1I Einhalskolben wurden 54,0 g (0,430 mol) 2,4,4-Trimethyl-2-cyclopenten-1-on (hergestellt gemäß H. E. Baumgarten (Hrsg.) Org. Synth. 5 (Coll.Vol.), 765, Wiley New York 1973) und 49,0 g (0,430) Selendioxid in 400 ml Eisessig für vier Stunden unter Rückfluß erhitzt. Es wurde noch heiß vom ausgefallenen, schwarzen Selen abfiltriert. Die Essigsäure wurde am Rotationsverdampfer abdestilliert, es blieb eine rot-braune, teilweise fest werdende Masse zurück, die in eine Kristallisierschale gegossen und über Nacht stehen gelassen wurde. Nach zweimaligem Umkristallisieren in Diethylether wurde das Produkt in Form eines hellorangen, kristallinen Pulvers erhalten.
- Ausbeute:: 11,8 g (0,090 mmol), 20% der Theorie
- Schmelzpunkt: 81 °C

### 2. Stufe: Darstellung von 5-Aza-6-methoxy-1,1,3,4-tetramethyl-1H-cyclopenta(b)-chinoxaliniumtetrafluoroborat

Es wurden 1,00 g (4,4 mmol) 3-Amino-2-methylamino-6-Methoxy-pyridin*2HCl zusammen mit 0,61 g (4,4 mmol) 3,5,5-Trimethyl-cyclopent-2-en-1,2-dion in 50 ml Methanol und 5 ml 32%iger Tetrafluorborsäure 6 Stunden bei Raumtemperatur gerührt. Der ausgefallene gelbbraune Niederschlag wurde über einen Büchnertrichter abfiltriert und mit wenig Methanol gewaschen. Es wurde zweimal in Aceton umkristallisiert. Das Produkt fiel in Form eines hellgrünen, kristallinen Feststoffes an.
- Ausbeute:: 150 mg (0,4 mmol), 10% der Theorie
- Schmelzpunkt: 275°C

### Herstellung der Färbegele

Die angegebene Menge der Cyclopentachinoxaliniumverbindung wurde in Wasser gelöst und die Lösung mit 2 % Natrosol®250 HR angedickt.

| Zusammensetzung der Färbegele (Tab. 1) | | | |
|---|---|---|---|
| Komponenten | Färbegel 1 Menge (g) | Färbegel 2 Menge (g) | Färbegel 3 Menge (g) |
| 5-Aza-6-methoxy-1,2,3,4-tetramethyl-1H-cyclopenta(b)chinoxaliniumtetrafluoroboranat | 6,84 | - | - |
| 1,1,2,3,4-Pentamethyl-1H-cyclopenta(b)chinoxaliniumtetrafluoroboranat | - | 6,52 | - |
| 5-Aza-6-methoxy-1,1,2,3,4-penta-methyl-1H-cyclopenta(b)chinoxalinium-tetrafluoroboranat | - | - | 7,14 |
| Natrosol® 250 HR | 2,0 | 2,0 | 2,0 |
| Wasser, dest. | ad 100 | ad 100 | ad 100 |

### Herstellung der Farbcremes

Texapon®NSO, Dehyton®K, Hydrenol®D, Lorol® techn. und Eumulgin®B 2 wurden unter Zusatz von ca. 10 g Wasser bei 80° C emulgiert. Separat wurden die Aldehyde in Wasser gelöst und die Lösung mit der Emulsion vermischt.

### Legende zu den Tabellen 1 und 2

- Natrosol®250 HR =: Hydroxyethylcellulose
- Texapon®NSO =: Na-Laurylethersulfat (26.5 -27%ig)
- Dehyton®K =: Cocoamidopropylbetain (29 - 32%ig)
- Hydrenol®D =: Cetearylalkohol
- Lorol®techn. =: Kokosfettalkohol
- Eumulgin®B2 =: Ceteareth-20

Die Färbegele 1 bis 3 wurden allein oder nach dem Vermischen mit einer der Farbcremes aus Tab. 2 (im Gew.-Verhältnis 1:1) eingesetzt. Vor der Applikation wurde der pH-Wert mit Ammoniak auf den in Tab. 3 angegebenen Wert eingestellt. Ausgefärbt wurde auf naturweißen Haarsträhnen der Fa. Kerling. Die Einwirkzeit betrug 30 Min. bei 32°C.

Es wurden die in Tabelle 3 genannte Farbergebnisse gemäß Deutschem Farbatlas erhalten.

**Tabelle 2**

| Komponenten | **A** | **B** | **C** | **D** |
|---|---|---|---|---|
| Texapon®NSO | 20,00 | 20,00 | 20,00 | 20,00 |
| Dehyton®K | 12,50 | 12,50 | 12,50 | 12,50 |
| Hydrenol®D | 8,50 | 8,50 | 8,50 | 8,50 |
| Lorol®, techn | 2,00 | 2,00 | 2,00 | 2,00 |
| Eumulgin® B2 | 0,75 | 0,75 | 0,75 | 0,75 |
| Vanillin | 3,04 | - | - | - |
| 4-(N,N-Dimethylamino)benzaldehyd | - | 3,00 | - | - |
| 4-Dimethylaminozimtaldehyd | - | - | 3,50 | - |
| 4-Dimethylamino-1-naphthaldehyd | - | - | - | 3,99 |
| Ammoniak, 25%ig | 4,80 | 5,00 | 5,20 | 5,00 |
| Wasser, dest. | ad 100 | ad 100 | ad 100 | ad 100 |
| **pH-Wert** | **9,03** | **9,48** | **9,40** | **9,39** |

**Tabelle 3**

| **Färbegel** | **Farbcreme** | **pH** | **Farbergebnis** |
|---|---|---|---|
| 1 | C | 6,5 | maisgelb |
| 1 | - | 6,4 | blassgelb |
| 1 | - | 9,7 | pastellgelb |
| 1 | C | 9,0 | buttergelb |
| 2 | - | 6,1 | rotbraun |
| 2 | - | 9,7 | mahagonibraun |
| 2 | A | 6,2 | violettbraun |
| 2 | A | 9,0 | madeirabraun |
| 2 | B | 5,9 | eichenbraun |
| 2 | B | 9,8 | helltopasgelb |
| 2 | C | 6,2 | orange |
| 2 | C | 9,5 | braunorange |
| 2 | D | 6,4 | grauviolett |
| 2 | D | 9,8 | graublau |
| 3 | - | 6,2 | blassgelb |
| 3 | - | 9,7 | giftgelb |
| 3 | B | 6,2 | knallgelb |
| 3 | B | 9,2 | knallgelb |
| 3 | C | 6,0 | maisgelb |
| 3 | C | 8,9 | maisgelb |

## Patentansprüche

1. Mittel zum Färben von keratinhaltigen Fasern, insbesondere menschlichen Haaren, enthaltend als färbende Komponente mindestens ein Cyclopentachinoxaliniumderivat mit der Formel I, 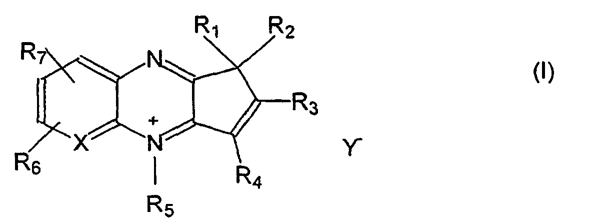 in der bedeuten
R¹, R², R³, R⁴, die jeweils gleich oder verschieden sein können, Wasserstoff oder C₁-C₄-Alkylgruppen,
R⁵ ein C₁-C₄-Alkyl-, ein Ar-C₁-C₄-alkyl-, Aryl-, C₂-C₄-Alkenyl-, C₁-C₄-Hydroxyalkyl- oder C₁-C₄-Carboxyalkylrest,
X eine Gruppe CH oder ein Stickstoffatom,
R⁶ und R⁷, die jeweils gleich oder verschieden sein können, Halogen, eine Hydroxyl-, Amino-, C₁-C₄-Alkylamino-, C₁-C₄-Alkoxy-, Nitro-, C₁-C₄-Alkyl-Gruppe, Wasserstoff, eine -COOH- oder ―SO₃H-Gruppe,
Y⁻ Halogenid, Benzolsulfonat, p-Toluolsulfonat, Methansulfonat, Methylsulfat, Ethylsulfat, Trifluormethansulfonat, Perchlorat, Sulfat, Hydrogensulfat, Tetrafluorborat, oder Tetrachlorzinkat.

2. Mittel nach Anspruch 1, **dadurch gekennzeichnet, dass** als Verbindungen mit der Formel I aus 5-Aza-6-methoxy-1,1,3,4-tetramethyl-1H-cyclopenta(b)chinoxalinium-, 5-Aza-6-methoxy-1,1,2,3,4-pentamethyl-1H-cyclopenta(b)chinoxalinium-, 5-Aza-1,1,2,3,4-pentamethyl-1H-cyclopenta(b)chinoxalinium- 1,1,2,3-Tetramethyl-1H-cyclopenta(b)chinoxalinium-, 4-Phenyl-1,1,2,3-tetramethyl-1H-cyclopenta(b)chinoxalinium-, 7-Chlor-4-(2-propyl)-1,1,2,3-Tetramethyl-1H-cyclopenta(b)chinoxalinium-, 7-Chlor-4-ethyl-1,1,2,3-Tetramethyl-1H-cyclopenta(b)chinoxalinium-, 1,1,2,3,4-Pentamethyl-1H-cyclopenta(b)chinoxalinium-halogenide, wie -chloride, -bromide und -iodide, -benzolsulfonate, p-toluolsulfonate, -methansulfonate, -methylsulfate, -ethylsulfate, -trifluormethansulfonate, -perchlorate, -sulfate, -hydrogensulfate, -tetrafluorborate, -tetrachlorzinkate sowie deren beliebigen Gemische eingesetzt werden.

3. Mittel nach einem der Ansprüche 1 bis 2, **dadurch gekennzeichnet, dass** die Cyclopentachinoxaliniumderivate der Formel I in einer Menge von 0,03 bis 65 mmol, insbesondere von 1 bis 40 mmol, bezogen auf 100 g des gesamten Färbemittels, enthalten sind.

4. Mittel nach einem der Ansprüche 1 bis 3, **dadurch gekennzeichnet, dass** es zusätzlich mindestens einen aromatischen/heteroaromatischen Aldehyd oder ein aromatisches/heteroaromatisches Keton enthält.

5. Mittel nach Anspruch 4, **dadurch gekennzeichnet, dass** die aromatischen/heteroaromatischen Aldehyde oder Ketone ausgewählt ist aus Salicylaldehyd, 3-Hydroxybenzaldehyd, 4-Hydroxybenzaldehyd, o-Anisaldehyd, m-Anisaldehyd, p-Anisaldehyd, 4-Hydroxy-3-methylbenzaldehyd, 2-Hydroxy-3-methylbenzaldehyd, 2-Hydroxy-5-methylbenzaldehyd, 2-Hydroxy-4-methylbenzaldehyd, 2,3-Dihydroxybenzaldehyd, 2,5- Dihydroxybenzaldehyd, 3,4-Dihydroxybenzaldehyd, 2,4- Dihydroxybenzaldehyd, 3,5- Dihydroxybenzaldehyd, 2,3-Dihydrobenzo[b]furan-5-carboxaldehyd, Piperonal, 4-Ethoxy-benzaldehyd, 3,5-Dimethyl-4-hydroxybenzaldehyd, Vanillin, Isovanillin, 2,3,4-Trihydroxybenzaldehyd, 2,4,5-Trihydroxybenzaldehyd, 2,4,6-Trihydroxybenzaldehyd, 3,4,5-Trihydroxybenzaldehyd, 3-Chlor-4-hydroxybenzaldehyd, 2,4-Dimethoxybenzaldehyd, 2,5-Dimethoxybenzaldehyd, 2,6-Dimethoxybenzaldehyd, 3,4-Dimethoxybenzaldehyd, 3,4-Dihydroxy-5-methoxybenzaldehyd, 2-Hydroxy-1-naphthaldehyd, 4-Hydroxy-1-naphthaldehyd, 1-Hydroxy-2-naphthaldehyd, 2-Methoxy-1-naphthaldehyd, 4-Methoxy-1-naphthaldehyd, 2-Hydroxyacetophenon, 4-Hydroxyacetophenon, 2,4-Dihydroxyacetophenon, 2-Hydroxyzimtaldehyd, 4-Hydroxyzimtaldehyd, 2,4-Dihydroxyzimtaldehyd, 4-Hydroxy-benzylidenaceton, 4-Hydroxy-3-methoxybenzylidenaceton, 4-Hydroxy-3-methoxyzimtaldehyd (Coniferylaldehyd), 3,5-Dimethoxy-4-hydroxyzimtaldehyd, 4-Hydroxycinnamylidenacetaldehyd, 4-Methoxycinnamylidenacetaldehyd sowie beliebigen Gemischen der voranstehenden.

6. Mittel nach einem der Ansprüche 1 bis 5, **dadurch gekennzeichnet, dass** es Farbverstärker ausgewählt aus der Gruppe bestehend aus Piperidin, Piperidin-2-carbonsäure, Piperidin-3-carbonsäure, Piperidin-4-carbonsäure, Pyridin, 2-Hydroxypyridin, 3-Hydroxypyridin, 4-Hydroxypyridin, Imidazol, 1-Methylimidazol, Histidin, Pyrrolidin, Prolin, Pyrrolidon, Pyrrolidon-5-carbonsäure, Pyrazol, 1,2,4-Triazol, Piperazidin oder deren beliebigen Gemischen enthält.

7. Mittel nach einem der Ansprüche 1 bis 6, **dadurch gekennzeichnet, dass** es direkt ziehende Farbstoffe aus der Gruppe der Nitrophenylendiamine, Nitroaminophenole, Anthrachinone oder Indophenole vorzugsweise in einer Menge von 0,01 bis 20 Gew.-%, bezogen auf das gesamte Färbemittel, enthält.

8. Mittel nach einem der Ansprüche 1 bis 7, **dadurch gekennzeichnet, dass** Ammonium- oder Metallsalze ausgewählt aus der Gruppe der Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, zugegeben werden.

9. Mittel nach einem der Ansprüche 1 bis 8, **dadurch gekennzeichnet, dass** es Oxidationsmittel, insbesondere H₂O₂, in einer Menge von 0,01 bis 6 Gew.-%, bezogen auf die Anwendungslösung, enthält.

10. Mittel nach einem der Ansprüche 1 bis 9, **dadurch gekennzeichnet, dass** es anionische, zwitterionische oder nichtionische Tenside enthält.

11. Verwendung von Cyclopentachinoxaliniumderivaten mit der Formel I, 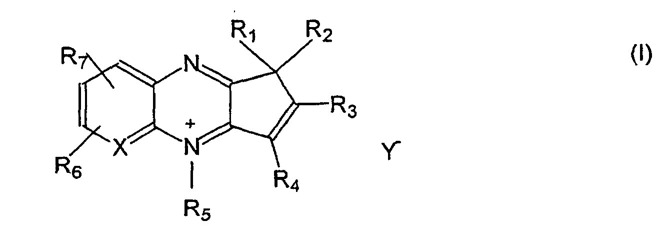 in der bedeuten
R¹, R², R³, R⁴, die jeweils gleich oder verschieden sein können, Wasserstoff oder C₁-C₄-Alkylgruppen,
R⁵ ein C₁-C₄-Alkyl-, ein Ar-C₁-C₄-alkyl-, Aryl-, C₂-C₄-Alkenyl-, C₁-C₄-Hydroxyalkyl- oder C₁-C₄-Carboxyalkylrest,
X, eineGruppe CH oder ein Stickstoffatom
R⁶ und R⁷, die jeweils gleich oder verschieden sein können, Halogen, eine Hydroxyl-, Amino-, C₁-C₄-Alkylamino-, C₁-C₄-Alkoxy-, Nitro-, C₁-C₄-Alkyl-Gruppe, Wasserstoff-, eine -COOH- oder -SO₃H-Gruppe,
Y⁻ Halogenid, Benzolsulfonat, p-Toluolsulfonat, Methansulfonat, Methylsulfat, Ethylsulfat, Trifluormethansulfonat, Perchlorat, Sulfat, Hydrogensulfat, Tetrafluorborat, oder Tetrachlorzinkat,
als eine färbende Komponente in Oxidationshaarfärbemitteln.

12. Verfahren zum Färben von keratinhaltigen Fasern, insbesondere menschlichen Haaren, worin ein Färbemittel, enthaltend
A) mindestens ein Cyclopentachinoxaliniumderivat mit der Formel I 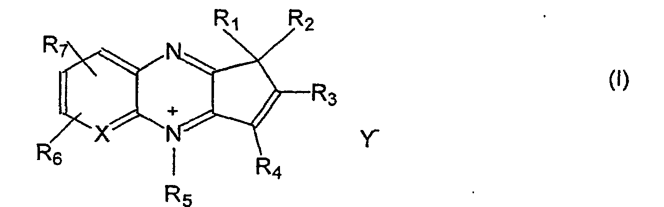 in der bedeuten
R¹, R², R³, R⁴, die jeweils gleich oder verschieden sein können, Wasserstoff oder C₁-C₄-Alkylgruppen,
R⁵ ein C₁-C₄-Alkyl-, ein Ar-C₁-C₄-alkyl-, Aryl-, C₂-C₄-Alkenyl-, C₁-C₄-Hydroxyalkyl- oder C₁-C₄-Carboxyalkylrest,
X, eine Gruppe CH oder ein Stickstoffatom
R⁶ und R⁷, die jeweils gleich oder verschieden sein können, Halogen, eine Hydroxyl-, Amino-, C₁-C₄-Alkylamino-, C₁-C₄-Alkoxy-, Nitro-, C₁-C₄-Alkyl-Gruppe, Wasserstoff-, eine -COOH- oder -SO₃H-Gruppe,
Y⁻ Halogenid, Benzolsulfonat, p-Toluolsulfonat, Methansulfonat, Methylsulfat, Ethylsulfat, Trifluormethansulfonat, Perchlorat, Sulfat, Hydrogensulfat, Tetrafluorborat, oder Tetrachlorzinkat,
und
B) einen oder mehrere aromatische/heteroaromatische Aldehyde und Ketone, sowie übliche kosmetische Inhaltsstoffe, auf die keratinhaltigen Fasern aufgebracht, einige Zeit, üblicherweise ca. 30 Minuten, auf der Faser belassen und anschließend wieder ausgespült oder mit einem Shampoo ausgewaschen wird.

13. 5-Aza-6-methoxy-1,1,3,4-tetramethyl-1H-cyclopenta(b)chinoxalinium-Salze.

14. 5-Aza-6-methoxy-1,1,2,3,4-pentamethyl-1H-cyclopenta(b)chinoxalinium-Salze.

15. Verbindung nach Anspruch 13 oder Anspruch 14, **dadurch gekennzeichnet, daß** die Salze ausgewählt sind aus den Halogeniden, wie Chloriden, Bromiden und Iodiden, Benzolsulfonaten, p-Toluolsulfonaten, Methansulfonaten, Methylsulfaten, Ethylsulfaten, Trifluormethansulfonaten, Perchloraten, Sulfaten, Hydrogensulfaten, Tetrafluorboraten und/oder Tetrachlorzinkaten.

## Claims

1. Preparation for colouring keratin-containing fibres, more particularly human hair, containing as colouring component at least one cyclopentaquinoxalinium derivative corresponding to formula I: 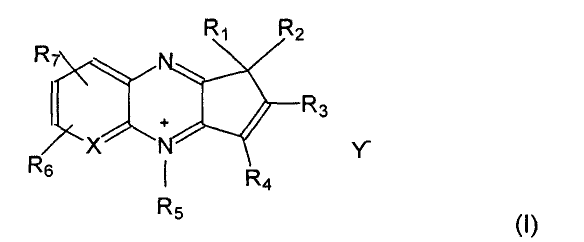 in which
R¹, R², R³ and R⁴ may be the same or different and represent hydrogen or C₁₋₄ alkyl groups,
R⁵ is a C₁₋₄ alkyl, an Ar C₁₋₄ alkyl, aryl, C₂₋₄ alkenyl, C₁₋₄ hydroxyalkyl or C₁₋₄ carboxyalkyl group,
X is a group CH or a nitrogen atom,
R⁶ and R⁷ may be the same or different and represent halogen, a hydroxyl, amino, C₁₋₄ alkylamino, C₁₋₄ alkoxy, nitro, C₁₋₄ alkyl group, hydrogen, a -COOH or an -SO₃H group,
Y⁻ is halide, benzenesulfonate, p-toluenesulfonate, methanesulfonate, methyl sulfate, ethyl sulfate, trifluoromethane sulfonate, perchlorate, sulfate, hydrogen sulfate, tetrafluoroborate or tetrachlorozincate.

2. Preparation as claimed in claim 1, **characterized in that** the compounds of formula (I) are selected from 5-aza-6-methoxy-1,1,3,4-tetramethyl-1H-cyclopenta(b)quinoxalinium-, 5-aza-6-methoxy-1,1,2,3,4-pentamethyl-1H-cyclopenta(b)quinoxalinium-, 5-aza-1,1,2,3,4-pentamethyl-1H-cyclopenta(b)quinoxalinium- 1,1,2,3-tetramethyl-1H-cyclopenta(b)quinoxalinium-, 4-phenyl-1,1,2,3-tetramethyl-1H-cyclopenta(b)quinoxalinium-, 7-chloro-4-(2-propyl)-1,1,2,3-tetramethyl-1H-cyclopenta(b)quinoxalinium-, 7-chloro-4-ethyl-1,1,2,3-tetramethyl-1H-cyclopenta(b)quinoxalinium-, 1,1,2,3,4-pentamethyl-1H-cyclopenta(b)quinoxalinium halides, such as chlorides, bromides and iodides, benzenesulfonates, p-toluenesulfonates, methanesulfonates, methyl sulfates, ethyl sulfates, trifluormethanesulfonates, perchlorates, sulfates, hydrogen sulfates, tetrafluoroborates, tetrachlorozincates and mixtures thereof.

3. Preparation as claimed in claim 1 or 2, **characterized in that** the cyclopentaquinoxalinium derivatives of formula (I) are present in a quantity of 0.03 to 65 mmol and more particularly 1 to 40 mmol, based on 100 g of the colorant as a whole.

4. Preparation as claimed in any of claims 1 to 3, **characterized in that** it additionally contains at least one aromatic/heteroaromatic aldehyde or an aromatic/heteroaromatic ketone.

5. Preparation as claimed in claim 4, **characterized in that** the aromatic/heteroaromatic aldehydes or ketones are selected from salicylaldehyde, 3-hydroxybenzaldehyde, 4-hydroxybenzaldehyde, o-anisaldehyde, m-anisaldehyde, p-anisaldehyde, 4-hydroxy-3-methylbenzaldehyde, 2-hydroxy-3-methylbenzaldehyde, 2-hydroxy-5-methylbenzaldehyde, 2-hydroxy-4-methylbenzaldehyde, 2,3-dihydroxybenzaldehyde, 2,5-dihydroxybenzaldehyde, 3,4-dihydroxybenzaldehyde, 2,4-dihydroxybenzaldehyde, 3,5-dihydroxybenzaldehyde, 2,3-dihydrobenzo[b]furan-5-carboxaldehyde, piperonal, 4-ethoxybenzaldehyde, 3,5-dimethyl-4-hydroxybenzaldehyde, vanillin, isovanillin, 2,3,4-trihydroxybenzaldehyde, 2,4,5-trihydroxybenzaldehyde, 2,4,6-trihydroxybenzaldehyde, 3,4,5-trihydroxybenzaldehyde, 3-chloro-4-hydroxybenzaldehyde, 2,4-dimethoxybenzaldehyde, 2,5-dimethoxybenzaldehyde, 2,6-dimethoxybenzaldehyde, 3,4-dimethoxybenzaldehyde, 3,4-dihydroxy-5-methoxybenzaldehyde, 2-hydroxy-1-naphthaldehyde, 4-hydroxy-1-naphthaldehyde, 1-hydroxy-2-naphthaldehyde, 2-methoxy-1-naphthaldehyde, 4-methoxy-1-naphthaldehyde, 2-hydroxyacetophenone, 4-hydroxyacetophenone, 2,4-dihydroxyacetophenone, 2-hydroxycinnamaldehyde, 4-hydroxycinnamaldehyde, 2,4-dihydroxycinnamaldehyde, 4-hydroxybenzylidene acetone, 4-hydroxy-3-methoxybenzylidene acetone, 4-hydroxy-3-methoxycinnamaldehyde (coniferyl aldehyde), 3,5-dimethoxy-4-hydroxycinnamaldehyde, 4-hydroxycinnamylidene acetaldehyde, 4-methoxycinnamylidene acetaldehyde and mixtures thereof.

6. Preparation as claimed in any of claims 1 to 5, **characterized in that** it contains colour intensifiers selected from the group consisting of piperidine, piperidine-2-carboxylic acid, piperidine-3-carboxylic acid, piperidine-4-carboxylic acid, pyridine, 2-hydroxypyridine, 3-hydroxypyridine, 4-hydroxypyridine, imidazole, 1-methylimidazole, histidine, pyrrolidine, proline, pyrrolidone, pyrrolidone-5-carboxylic acid, pyrazole, 1,2,4-triazole, piperazidine or mixtures thereof.

7. Preparation as claimed in any of claims 1 to 6, **characterized in that** it contains substantive dyes from the group of nitrophenylenediamines, nitroaminophenols, anthraquinones or indophenols, preferably in a quantity of 0.01 to 20% by weight, based on the colorant as a whole.

8. Preparation as claimed in any of claims 1 to 7, **characterized in that** ammonium or metal salts selected from the group of formates, carbonates, halides, sulfates, butyrates, valerates, caproates, acetates, lactates, glycolates, tartrates, citrates, gluconates, propionates, phosphates and phosphonates of alkali metals, such as potassium, sodium or lithium, alkaline earth metals, such as magnesium, calcium, strontium or barium, or of aluminium, manganese, iron, cobalt, copper or zinc are added.

9. Preparation as claimed in any of claims 1 to 8, **characterized in that** it contains oxidizing agents, more particularly H₂O₂, in a quantity of 0.01 to 6% by weight, based on the solution applied.

10. Preparation as claimed in any of claims 1 to 9, **characterized in that** it contains anionic, zwitterionic or nonionic surfactants.

11. Use of cyclopentaquinoxalinium derivatives corresponding to formula (I): 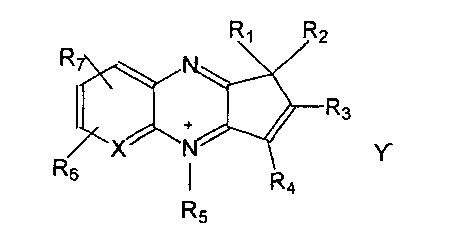 in which
R¹, R², R³ and R⁴ may be the same or different and represent hydrogen or C₁₋₄ alkyl groups,
R⁵ is a C₁₋₄ alkyl, an Ar C₁₋₄ alkyl, aryl, C₂₋₄ alkenyl, C₁₋₄ hydroxyalkyl or C₁₋₄ carboxyalkyl group,
X is a group CH or a nitrogen atom,
R⁶ and R⁷ may be the same or different and represent halogen, a hydroxyl, amino, C₁₋₄ alkylamino, C₁₋₄ alkoxy, nitro, C₁₋₄ alkyl group, hydrogen, a -COOH or an ―SO₃H group,
Y⁻ is halide, benzenesulfonate, p-toluenesulfonate, methanesulfonate, methyl sulfate, ethyl sulfate, trifluoromethane sulfonate, perchlorate, sulfate, hydrogen sulfate, tetrafluoroborate or tetrachlorozincate,
as a colouring component in oxidation hair colorants.

12. A process for colouring keratin fibres, more particularly human hair, in which a colorant containing
A) at least one cyclopentaquinoxalinium derivative corresponding to formula (I): 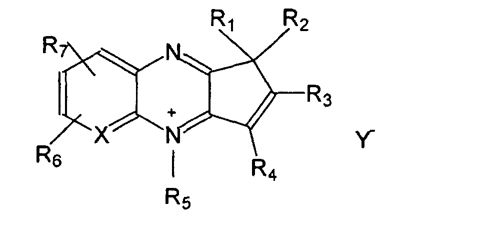 in which
R¹, R², R³ and R⁴ may be the same or different and represent hydrogen or C₁₋₄ alkyl groups,
R⁵ is a C₁₋₄ alkyl, an Ar C₁₋₄ alkyl, aryl, C₂₋₄ alkenyl, C₁₋₄ hydroxyalkyl or C₁₋₄ carboxyalkyl group,
X is a group CH or a nitrogen atom,
R⁶ and R⁷ may be the same or different and represent halogen, a hydroxyl, amino, C₁₋₄ alkylamino, C₁₋₄ alkoxy, nitro, C₁₋₄ alkyl group, hydrogen, a -COOH or an -SO₃H group,
Y⁻ is halide, benzenesulfonate, p-toluenesulfonate, methanesulfonate, methyl sulfate, ethyl sulfate, trifluoromethane sulfonate, perchlorate, sulfate, hydrogen sulfate, tetrafluoroborate or tetrachlorozincate,
and
B) one or more aromatic/heteroaromatic aldehyde and ketones and typical cosmetic ingredients is applied to the keratin-containing fibres, left on the fibres for a while, typically for about ca. 30 minutes, and then rinsed out again or washed out with a shampoo.

13. 5-Aza-6-methoxy-1,1,3,4-tetramethyl-1H-cyclopenta(b)quinoxalinium salts.

14. 5-Aza-6-methoxy-1,1,2,3,4-pentamethyl-1H-cyclopenta(b)quinoxalinium salts.

15. Compound as claimed in claim 13 or claim 14, **characterized in that** the salts are selected from the halides, such as chlorides, bromides and iodides, benzenesulfonates, p-toluenesulfonates, methanesulfonates, methyl sulfates, ethyl sulfates, trifluormethanesulfonates, perchlorates, sulfates, hydrogen sulfates, tetrafluoroborates and/or tetrachlorozincates.

## Revendications

1. Agent pour la teinture de fibres kératinisées, en particulier de cheveux humains, contenant, à titre de composant de teinture, au moins un dérivé de cyclopentaquinoxalinium répondant à la formule I 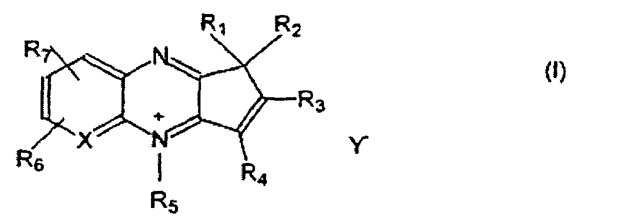 dans laquelle
R¹, R², R³, R⁴, qui peuvent être identiques ou différents l'un de l'autre, représentent un atome d'hydrogène ou un groupe alkyle en C₁-C₄ ;
R⁵ représente un radical alkyle en C₁-C₄, un radical aralkyle en C₁-C₄, un radical aryle, un radical alcényle en C₂-C₄, un radical hydroxyalkyle en C₁-C₄ ou un radical carboxyalkyle en C₁-C₄ ;
X représente un groupe CH ou un atome d'azote ;
R⁶ et R⁷, qui peuvent être identiques ou différents l'un de l'autre représentent un atome d'halogène, un groupe hydroxyle, un groupe amino, un groupe alkyl(en C₁-C₄)amino, un groupe alcoxy en C₁-C₄, un groupe nitro, un groupe alkyle en C₁-C₄, un atome d'hydrogène, un groupe -COOH- ou un groupe -SO₃H- ;
Y⁻ représente un halogénure, un benzènesulfonate, un p-toluènesulfonate, un méthanesulfonate, un méthylsulfate, un éthylsulfate, un trifluorométhanesulfonate, un perchlorate, un sulfate, un hydrogénosulfate, un tétrafluoroborate ou un tétrachlorozincate.

2. Agent selon la revendication 1, **caractérisé en ce qu'**on met en oeuvre, à titre de composés répondant à la formule 1, un membre choisi parmi le groupe comprenant des halogénures tels que des chlorures, des bromures et des iodures, des benzènesulfonates, des p-toluènesulfonates, des méthanesulfonates, des méthylsulfates, des éthylsulfates, des trifluorométhanesulfonates, des perchlorates, des sulfates, des hydrogénosulfates, des tétrafluoroborates, des tétrachlorozincates du 5-aza-6-méthoxy-1,1,3,4-tétraméthyl-1H-cyclopenta(b)quinoxalinium, du 5-aza-6-méthoxy-1,1,2,3,4-pentaméthyl-1H-cyclopenta(b)quinoxalinium, du 5-aza-1,1,2,3,4-pentaméthyl-1H-cyclopenta(b)quinoxalinium, du 1,1,2,3-tétraméthyl-1H-cyclopenta(b)quinoxalinium, du 4-phényl-1,1,2,3-tétraméthyl-1H-cyclopenta(b)quinoxalinium, du 7-chloro-4-(2-propyl)-1,1,2,3-tétraméthyl-1H-cyclopenta(b)quinoxalinium, du 7-chloro-4-éthyl-1,1,2,3-tétraméthyl-1H-cyclopenta(b)quinoxalinium, du 1,1,2,3,4-pentaméthyl-1H-cyclopenta(b)quinoxalinium ou l'un quelconque de leurs mélanges.

3. Agent selon l'une quelconque des revendications 1 à 2, **caractérisé en ce qu'**il contient les dérivés de cyclopentaquinoxalinium répondant à la formule I en une quantité de 0,03 à 65 millimoles, en particulier de 1 à 40 millimoles, rapportés à 100 g de la totalité de l'agent de teinture.

4. Agent selon l'une quelconque des revendications 1 à 3, **caractérisé en ce qu'**il contient en outre au moins un aldéhyde aromatique/hétéroaromatique ou une cétone aromatique/hétéroaromatique.

5. Agent selon la revendication 4, **caractérisé en ce que** les aldéhydes ou les cétones aromatiques/hétéroaromatiques sont choisis parmi le groupe comprenant le salicylaldéhyde, le 3-hydroxybenzaldéhyde, le 4-hydroxybenzaldéhyde, le o-anisaldéhyde, le m-anisaldéhyde, le p-anisaldéhyde, le 4-hydroxy-3-méthylbenzaldéhyde, le 2-hydroxy-3-méthylbenzaldéhyde, le 2-hydroxy-5-méthylbenzaldéhyde, le 2-hydroxy-4-méthylbenzaldéhyde, le 2,3-dihydroxybenzaldéhyde, le 2,5-dihydroxybenzaldéhyde, le 3,4-dihydroxybenzaldéhyde, le 2,4-dihydroxybenzaldéhyde, le 3,5-dihydroxybenzaldéhyde, le 2,3-dihydrobenzo[b]furanne-5-carboxaldéhyde, le pipéronal, le 4-éthoxybenzaldéhyde, le 3,5-diméthyl-4-hydroxybenzaldéhyde, la vanilline, l'isovanilline, le 2,3,4-trihydroxybenzaldéhyde, le 2,4,5-trihydroxybenzaldéhyde, le 2,4,6-trihydroxybenzaldéhyde, le 3,4,5-trihydroxybenzaldéhyde, le 3-chloro-4-hydroxybenzaldéhyde, le 2,4-diméthoxybenzaldéhyde, le 2,5-diméthoxybenzaldéhyde, le 2,6-diméthoxybenzaldéhyde, le 3,4-diméthoxybenzaldéhyde, le 3,4-dihydroxy-5-méthoxybenzaldéhyde, le 2-hydroxy-1-naphtaldéhyde, le 4-hydroxy-1-naphtaldéhyde, le 1-hydroxy-2-naphtaldéhyde, le 2-méthoxy-1-naphtaldéhyde, le 4-méthoxy-1-naphtaldéhyde, la 2-hydroxyacétophénone, la 4-hydroxyacétophénone, la 2,4-dihydroxyacétophénone, le 2-hydroxycinnamaldéhyde, le 4-hydroxycinnamaldéhyde, le 2,4-dihydroxycinnamaldéhyde, la 4-hydroxybenzylidène-acétone, la 4-hydroxy-3-méthoxybenzylidène-acétone, le 4-hydroxy-3-méthoxycinnamaldéhyde (coniférylaldéhyde), le 3,5-diméthoxy-4-hydroxycinnamaldéhyde, le 4-hydroxycinnamylidèneacétaldéhyde, le 4-méthoxycinnamylidène-acétaldéhyde, ainsi que l'un quelconque de leurs mélanges.

6. Agent selon l'une quelconque des revendications 1 à 5, **caractérisé en ce qu'**il contient un renforçateur des couleurs choisi parmi le groupe constitué par la pipéridine, l'acide pipéridine-2-carboxylique, l'acide pipéridine-3-carboxylique, l'acide pipéridine-4-carboxylique, la pyridine, la 2-hydroxypyridine, la 3-hydroxypyridine, la 4-hydroxypyridine, l'imidazole, le 1-méthylimidazole, l'histidine, la pyrrolidine, la proline, la pyrrolidone, l'acide pyrrolidone-5-carboxylique, le pyrazole, le 1,2,4-triazole, la pipérazidine ou l'un quelconque de leurs mélanges.

7. Agent selon l'une quelconque des revendications 1 à 6, **caractérisé en ce qu'**il contient des colorants montant directement sur la fibre, choisis parmi le groupe comprenant des nitrophénylènediamines, des nitroaminophénols, des anthraquinones ou des indophénols, de préférence en une quantité de 0,01 à 20 % en poids, rapportés à la totalité de l'agent de teinture.

8. Agent selon l'une quelconque des revendications 1 à 7, **caractérisé en ce qu'**on ajoute des sels d'ammonium ou des sels métalliques choisis parmi le groupe des formiates, des carbonates, des halogénures, des sulfates, des butyrates, des valérates, des caproates, des acétates, des lactates, des glycollates, des tartrates, des citrates, des gluconates, des propionates, des phosphates et des phosphonates de métaux alcalins tels que le potassium, le sodium ou le lithium, de métaux alcalino-terreux tels que le magnésium, le calcium, le strontium ou le baryum, ou encore de l'aluminium, du manganèse, du fer, du cobalt, du cuivre ou du zinc.

9. Agent selon l'une quelconque des revendications 1 à 8, **caractérisé en ce qu'**il contient des agents d'oxydation, en particulier du H₂O₂, en une quantité de 0,01 à 6 % en poids rapportés à la solution d'utilisation.

10. Agent selon l'une quelconque des revendications 1 à 9, **caractérisé en ce qu'**il contient des agents tensioactifs anioniques, amphotères ou non ioniques.

11. Utilisation de dérivés de cyclopentaquinoxalinium répondant à la formule I 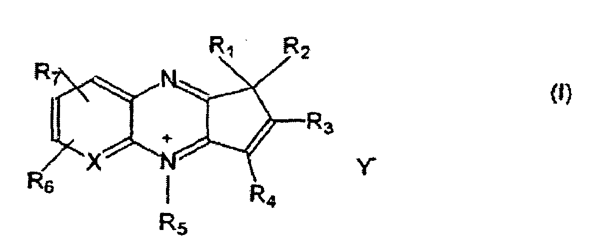 dans laquelle
R¹, R², R³, R⁴, qui peuvent être identiques ou différents l'un de l'autre, représentent un atome d'hydrogène ou un groupe alkyle en C₁-C₄ ;
R⁵ représente un radical alkyle en C₁-C₄, un radical aralkyle en C₁-C₄, un radical aryle, un radical alcényle en C₂-C₄, un radical hydroxyalkyle en C₁-C₄ ou un radical carboxyalkyle en C₁-C₄ ;
X représente un groupe CH ou un atome d'azote ;
R⁶ et R⁷, qui peuvent être identiques ou différents l'un de l'autre représentent un atome d'halogène, un groupe hydroxyle, un groupe amino, un groupe alkyl(en C₁-C₄)amino, un groupe alcoxy en C₁-C₄, un groupe nitro, un groupe alkyle en C₁-C₄, un atome d'hydrogène, un groupe -COOH- ou un groupe -SO₃H- ;
Y⁻ représente un halogénure, un benzènesulfonate, un p-toluènesulfonate, un trifluorométhanesulfonate, un perchlorate, un sulfate, un hydrogénosulfate, un tétrafluoroborate ou un tétrachlorozincate,
à titre de composant de teinture dans des teintures capillaires par oxydation.

12. Procédé pour la teinture de fibres kératinisées, en particulier de cheveux humains, dans lequel un agent de teinture, contenant
A) au moins un dérivé de cyclopentaquinoxalinium répondant à la formule I 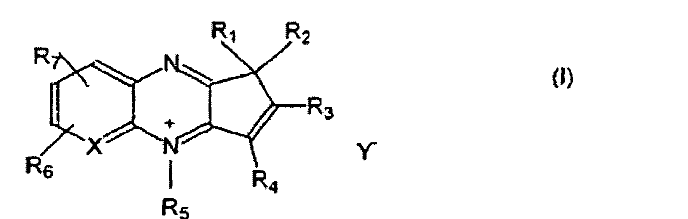 dans laquelle
R¹, R², R³, R⁴, qui peuvent être identiques ou différents l'un de l'autre, représentent un atome d'hydrogène ou un groupe alkyle en C₁-C₄ ;
R⁵ représente un radical alkyle en C₁-C₄, un radical aralkyle en C₁-C₄, un radical aryle, un radical alcényle en C₂-C₄, un radical hydroxyalkyle en C₁-C₄ ou un radical carboxyalkyle en C₁-C₄ ;
X représente un groupe CH ou un atome d'azote ;
R⁶ et R⁷, qui peuvent être identiques ou différents l'un de l'autre représentent un atome d'halogène, un groupe hydroxyle, un groupe amino, un groupe alkyl(en C₁-C₄)amino, un groupe alcoxy en C₁-C₄, un groupe nitro, un groupe alkyle en C₁-C₄, un atome d'hydrogène, un groupe -COOH- ou un groupe -SO₃H- ;
Y⁻ représente un halogénure, un benzènesulfonate, un p-toluènesulfonate, un méthanesulfonate, un méthylsulfate, un éthylsulfate, un trifluorométhanesulfonate, un perchlorate, un sulfate, un hydrogénosulfate, un tétrafluoroborate ou un tétrachlorozincate,
et
B) un ou plusieurs aldéhydes et cétones aromatiques/hétéroaromatiques, ainsi que des constituants cosmétiques habituels est appliqué sur les fibres kératinisées, laissé agir sur les fibres pendant un certain temps, habituellement pendant environ 30 minutes, avant d'être éliminé à nouveau par rinçage ou par lavage avec un shampooing.

13. Sels du 5-aza-6-méthoxy-1,1,3,4-tétraméthyl-1H-cyclopenta(b)-quinoxalinium.

14. Sels du 5-aza-6-méthoxy-1,1,2,3,4-pentaméthyl-1H-cyclopenta(b)-quinoxalinium.

15. Composé selon la revendication 13 ou 14, **caractérisé en ce que** les sels sont choisis parmi le groupe comprenant des halogénures telles que des chlorures, des bromures et des iodures, des benzènesulfonates, des p-toluènesulfonates, des méthanesulfonates, des méthylsulfates, des éthylsulfates, des trifluorométhanesulfonates, des perchlorates, des sulfates, des hydrogénosulfates, des tétrafluoroborates et/ou des tétrachlorozincates.
